# EUROPÄISCHE PATENTSCHRIFT

(11) **EP 0 522 256 B1**
(45) Veröffentlichungstag und Bekanntmachung des Hinweises auf die Patenterteilung: **30.06.1999**
(21) Anmeldenummer: 92107847.3
(22) Anmeldetag: 08.05.1992
(51) Int. Cl.: G01N 33/49, G01N 33/483, G01N 35/02

(54) **Einrichtung zur automatischen Untersuchung von Blutproben**
Apparatus for the automatic analysis of blood samples
Appareil pour l'analyse automatique des échantillons de sang

(30) Priorität: 28.05.1991 DE 4117482; 26.03.1992 DE 4209871
(43) Veröffentlichungstag der Anmeldung: 13.01.1993
(73) Patentinhaber: BAXTER DIAGNOSTICS INC., Deerfield, IL 60015 (US)
(72) Erfinder: Kratzer, Michael, Dr., D-8000 München 40 (DE); Freiherr von der Goltz, Volker, D-8221 Seeon (DE)
(74) Vertreter: von Puttkamer, Nikolaus, Dipl.-Ing. Patentanwälte Haft, von Puttkamer Berngruber, Czybulka

(56) Entgegenhaltungen:
- EP-A- 0 111 942
- EP-A- 0 335 789
- DE-A- 3 839 896
- US-A- 4 855 109

## Beschreibung

Die Erfindung betrifft eine Einrichtung zur automatischen Untersuchung von Blutproben.

Es ist aus der Druckschrift DE-A-3 541 057 bekannt, die Blutungszeit in-vitro dadurch zu messen, daß gemäß Figur 15 Blut 9' aus einem Vorratsgefäß 7' über eine Apertur 5' in einen Zylinder 6' dadurch eingesaugt wird, daß im Zylinder 6' ein Kolben 3' durch einen Schrittmotor 2' in der Richtung des Pfeiles 11' bewegt wird. Ein Drucksensor 4' mißt dabei den Druck, der in dem dem Kolben 3' vorgelagerten Raum herrscht. Dieser Druck wird dadurch auf einen konstanten Wert gehalten, daß ein Prozessor 1' den Schrittmotor 2' in Abhängigkeit vom Signal des Drucksensors 4' ansteuert. Aus der Bewegung des Kolbens 3' und dem Durchmesser des Zylinders 6' errechnet der Prozessor 1' den Volumenstrom des Blutes durch die Apertur 5'. Die Apertur 5', deren Durchmesser beispielsweise bei etwa 150 µm liegt, bildet einen verletzten Teil einer durchschnittenen Arteriole nach. Sie befindet sich beispielsweise in einem Zelluloseacetat-Filter der mit Kollagen beschichtet ist. Der Filter wird vor der Messung mit ADP getränkt. Nach dem beschriebenen Verfahren ist eine reproduzierbare Messung der Blutungszeit in vitro und des Blutungsvolumens möglich.

Bekannterweise ist die Apertur 5', d.h. also das genannte Filter in einem Aperturhalter 10' angeordnet, der mit einer Kapillare 8' verbindbar ist, über die das dem Vorratsgefäß 7' entnommenen Blut 9' zur Apertur 5' gesaugt wird. Nach dem bekannten Verfahren sind keine automatischen Untersuchungen von Blutproben möglich.

Ein Problem besteht bei dem beschriebenen Meßverfahren darin, daß bei Durchführung einer großen Anzahl von Messungen, beispielsweise von etwa 150 Messungen pro Tag, eine entsprechend große Anzahl von Aperturhaltern 10' erforderlich sind. Diese Aperturhalter 10', die die Apertur 5' und die beschriebenen kollagenbeschichteten Filter enthalten, müssen bis zu ihrem Gebrauch in einem Kühlschrank oder dergleichen bei etwa 4 °C gelagert werden. Dies bedeutet, daß bei der Durchführung von Messungen im großen Stil die zur Bereitstellung der Aperturhalter 10' erforderlichen, gekühlten Lagerräume sehr groß sein müssen.

Aus der Druckschrift EP-A-0 111 942 geht eine weitere Durchflußvorrichtung zur Messung des Blutungszeit hervor, die ebenfalls nicht für die automatische Untersuchung von Blutproben geeignet ist.

Die Aufgabe der vorliegenden Erfindung besteht daher darin, eine Einrichtung zur automatischen Untersuchung von Blutproben zuschaffen.

Diese Aufgabe wird durch eine Einrichtung zur automatischen Untersuchung von Blutproben mit den Merkmalen des Patentanspruches 1 gelöst.

Der wesentliche Vorteil der erfindungsgemäßen Einrichtung besteht darin, daß sie automatische Untersuchungen von Blutproben ermöglicht. Zudem sind in einem Magazin angeordnete Aperturhalter verwendbar, zu deren Speicherung bzw. Lagerung extrem wenig Raum erforderlich ist. Dies führt vorteilhafterweise dazu, daß in einer vorhandenen Kühleinrichtung sehr viel mehr Aperturhalter gelagert und gekühlt werden können, als dies bisher möglich ist. Dadurch kann neben der Einsparung von Lagerraum gleichzeitig auch Energie eingespart werden.

Da bei einer bevorzugten Ausführungsform der Erfindung der Prüfkopf und die Aperturhalter so ausgebildet sind, daß bei einer einzigen Meßoperation mehrere, z.B. zwei mal zwei Untersuchungen gleichzeitig durchgeführt werden können, kann die zuvor genannte Raum- und Energieeinsparung noch wesentlich erhöht werden. '

Weitere vorteilhafte Ausgestaltungen der Erfindung gehen aus Unteransprüchen hervor.

Im folgenden werden die Erfindung und deren Ausgestaltungen im Zusammenhang mit den Figuren näher erläutert. Es zeigen:
- Fig. 1: in schematischer Darstellung eine erfindungsgemäße Einrichtung zur automatischen Untersuchung von Blutproben;
- Fig. 2: eine Detaildarstellung des Prüfkopfes der erfindungsgemäßen Einrichtung;
- Fig. 3: eine Ansicht auf einen Aperturhalter mit einer Apertur;
- Fig. 4: eine Ansicht entlang der Linie IV-IV der Figur 2 auf einen Aperturhalter mit mehreren Aperturen;
- Fig. 5: die Aufsicht V-V auf den Kapillarenhalter des Prüfkopfes der Figur 2;
- Fig. 6, 7: Transporteinrichtungen für Aperturhalter und

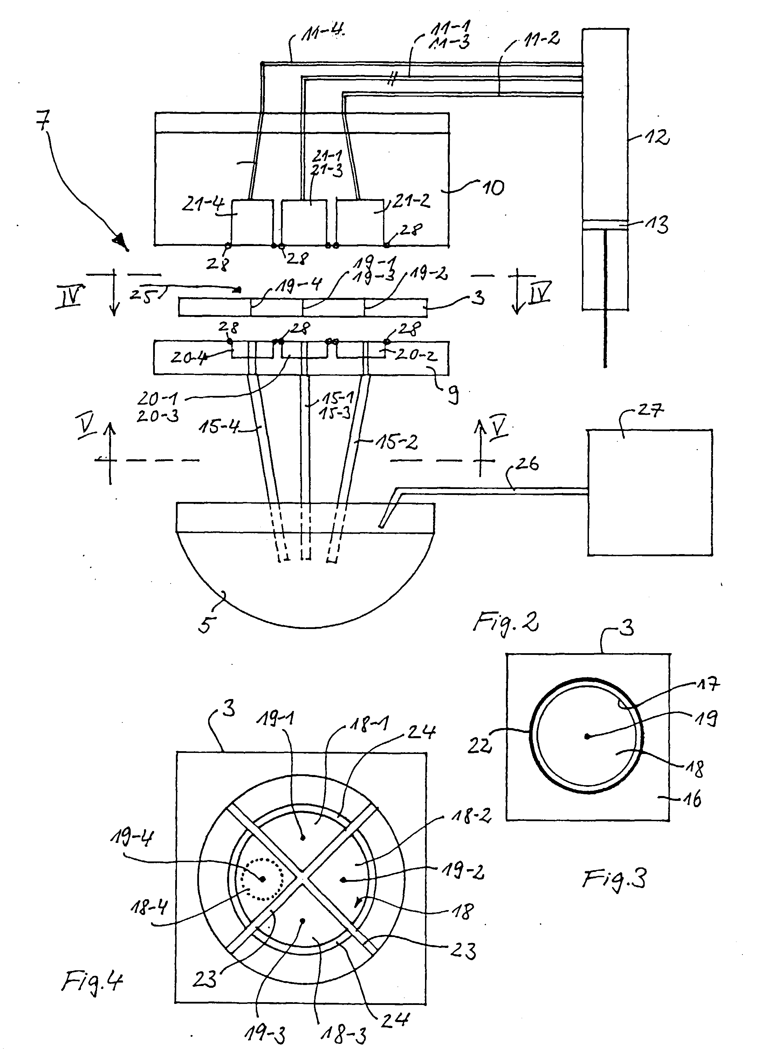

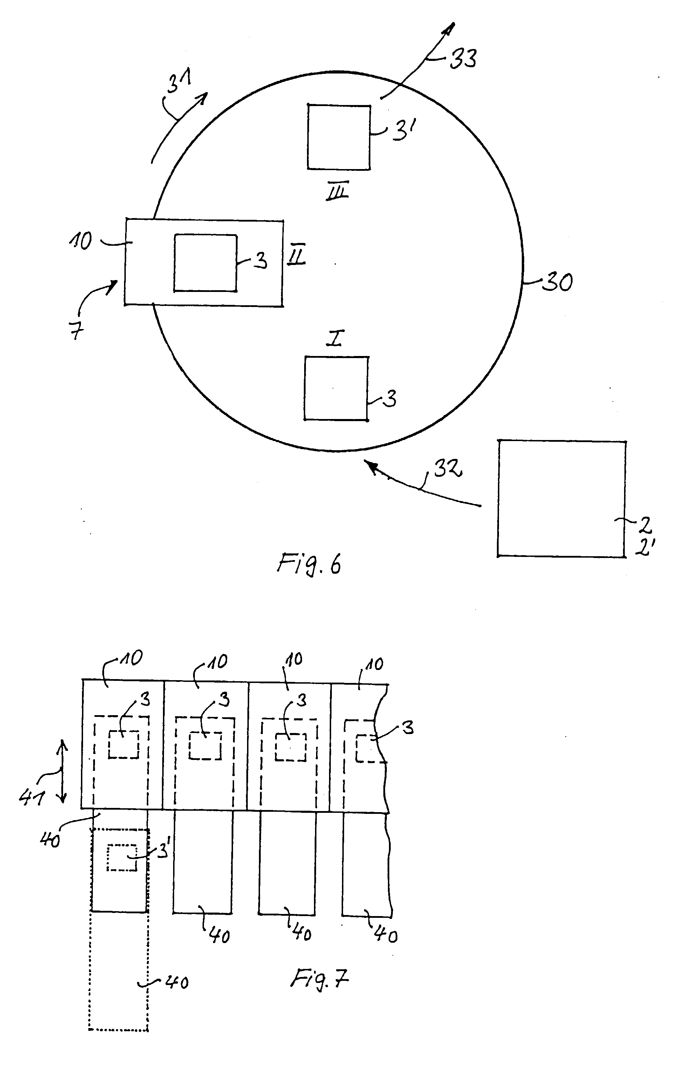

- Fig. 8: eine Darstellung zur Erläuterung des Standes der Technik.

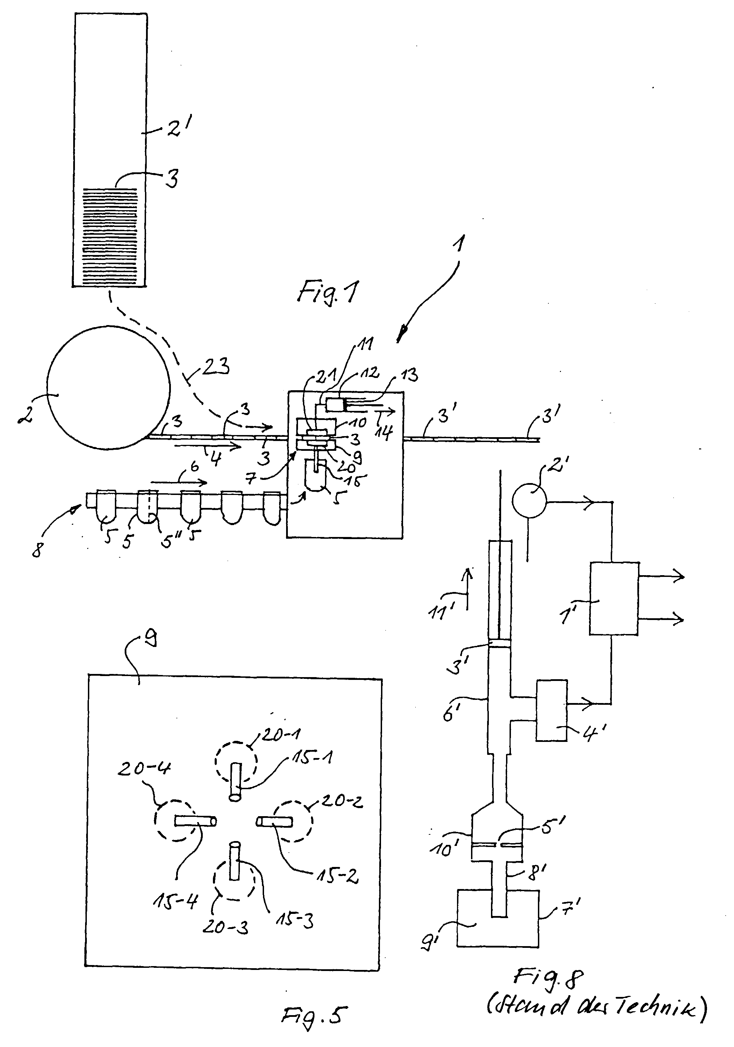

Aus der Prinzipdarstellung der Figur 1 ist ersichtlich, daß die vorliegende Untersuchungseinrichtung im wesentichen eine Prüfstation 1 aufweist, der von einer Magazineinrichtung 2,2' aufeinanderfolgend Aperturhalter 3 in der Richtung des Pfeiles 4 oder 230 zugeführt werden.

Der Prüfstation 1 werden ferner die zu untersuchenden Blutproben, die sich in Vorratsgefäßen 5 befinden, in der Richtung des Pfeiles 6 aufeinanderfolgend derart zugeführt, daß zu dem Prüfkopf 7 der Prüfstation 1 jeweils gleichzeitig ein Aperturhalter 3 und ein Vorratsgefäß 5 zugeführt werden. Dabei wird das Vorratsgefäß 5 vorzugsweise einer Magazin einrichtung 8 entnommen und zur Durchmischung der in ihm enthaltenen Blutprobe vor Eingabe in den Prüfkopf 7 mehrere Male invertiert. Die Entnahme des Vorratsgefäßes 5 aus dem Magazin 8 und die Invertierung des Vorratsgefäßes 5 erfolgen mit der Hilfe bekannter Vorrichtungen, die im einzelnen nicht näher erläutert werden. Der Prüfkopf 7 besteht im wesentlichen aus einem Kapillarenhalter 9, an dem eine Kapillare 15 befestigt ist, und einem Kopfteil 10, das über eine Leitung 11 mit einem Zylinder 12 verbunden ist, in dem ein Kolben 13 angeordnet ist. Bei der Bewegung des Kolbens 13 im Zylinder 12 in der Richtung des Pfeiles 14 wird in der Leitung 11 und dem Prüfkopf 7 ein Unterdruck erzeugt, der bewirkt, daß aus dem Vorratsgefäß 5, in das die Kapillare 15 gerade eintaucht, Blut entnommen und durch die Apertur des Aperturhalters 3 gesaugt wird.

Nach der Vornahme der einzelenen Messungen verlassen die gebrauchten Aperturhalter 3' die Prüfstation 1 als Wegwerfteile.

Gemäß Figur 3 weist ein Aperturhalter 3 beispielsweise die Form einer rechteckigen Platte 16 auf, die hinter einer Öffnung 17 ein scheibenförmiges Filterteil 18 hält, in der sich eine Apertur 19 befindet. Besonders vorteilhaft besteht der Aperturhalter 3 aus zwei deckungsgleich zueinander angeordneten Platten 16, von denen jede eine Öffnung 17 aufweist, so daß das scheibenförmige Filterteil 18, das beispielsweise aus Zelluloseacetat besteht und mit Kollagen beschichtet ist, zwischen den Platten 16 gehalten wird.

Beispielsweise sind die aus Kunststoff bestehenden Platten 16 aneinander verklebt oder verschweißt.

Zur Vornahme einer Messung werden der Kapillarenhalter 9 und das Kopfteil 10 derart auseinandergefahren, daß ein Aperturhalter 3 auf die Fläche des Kapillarenhalters 9 aufgelegt werden kann. Danach werden der Kapillarenhalter 9 und das Kopfteil 10 derart aufeinanderzubewegt, daß der Aperturhalter 3 zwischen den einander zugewandten Flächen des Kapillarenhalters 9 und des Kopfteiles 10 gehalten wird, so daß ein mit der Kapillare 15 in Verbindung stehender Raum des Kapillarenhalters 9 und ein mit der Leitung 11 verbundener Raum des Kopfteiles 10 unterhalb bzw. oberhalb des Filterteiles 18 angeordnet und zur Seite hin abgedichtet sind. Die genannten Räume werden vorzugsweise durch Vertiefungen 20 bzw. 21 gebildet, die in dem Kapillarenhalter 9 bzw. in dem Kopfteil 10 ausgebildet sind. Zur Bewirkung der Abdichtung kann, wie dies in der Figur 3 schematisch dargestellt ist, an beiden Seiten des Aperturhalters 3 eine die Öffnung 17 ringförmig umgebende Dichtung seinrichtung 22 vorgesehen sein, deren Durchmesser größer ist als der Durchmesser der Vertiefung 20 oder 21.

Nach dem Schließen des Prüfkopfes 7 durch Andrücken des Kapillarenhalters 9 und des Kopfteiles 10 gegen den Aperturhalter 3 kann die eigentliche Messung eingeleitet werden, da, wie dies bereits erläutert wurde, dafür Sorge getragen wird, daß sich nach dem Schließen des Prüfkopfes 7 unterhalb des Kapillarenhalters 9 ein Vorratsgefäß 5 derart befindet, daß die Kapillare 15 des Kapillarenhalters 9 in das Vorratsgefäß 5 eintaucht.

Aus der Figur 1 ist ersichtlich, daß die Magazineinrichtung 2 beispielsweise eine Vorratsrolle sein kann, von der die zu einem Band verbundenen Aperturhalter 3 abgewickelt werden. Die bandförmig auf die Vorratsspule aufgewickelten Aperturhalter 3 sind sehr kompakt, so daß sie bei ihrer Lagerung relativ wenig Raum benötigen.

An der Stelle der Vorratsrolle kann die Magazineinrichtung 2' beispielsweise auch die Form eines hohlzylinderförmigen Magazines aufweisen, in dem die einzelnen Aperturhalter 3 übereinander gestapelt sind. Aus dieser Magazineinrichtung 2' werden die Aperturhalter 3 aufeinanderfolgend in der Richtung des Pfeiles 230 mit der Hilfe bekannter mechanischer Vorrichtungen der Prüfstation 1 und dem Prüfkopf 7 zugeführt.

Durch eine ebenfalls nicht näher erläuterte Synchronisiereinrichtung wird dafür Sorge getragen, daß jeweils gleichzeitig ein Aperturhalter 3 aus der Magazineinrichtung 2, 2' und ein Vorratsgefäß 5 aus der Magazineinrichtung 8 dem Prüfkopf 7 zugeführt werden.

Im folgenden wird nun im Zusammenhang mit der Figur 2 eine besonders bevorzugte Ausgestaltung eines Prüfkopfes 7 näher erläutert. Im Gegensatz zu dem im Zusammenhang mit der Figur 1 beschriebenen Prüfkopf ist der Prüfkopf 7 der Figur 2 für Aperturhalter mit mehreren Aperturen, z.B. mit vier Aperturen 19-1, 19-2, 19-3 und 19-4 gedacht. Diese Aperturen sind vorzugsweise gleichmäßig auf der in dem Aperturhalter 3 gehaltenen Filterteil 18 verteilt. Beispielsweise sind gemäß Figur 4 die vier Aperturen 19-1 bis 19-4 gleichmäßig, um 90 ° voneinander beabstandet auf einem Kreis um den Mittelpunkt des Filterteiles 18 verteilt. Um eine Abdichtung der einzelnen Aperturen 19-1 bis 19-4 voneinander zu bewirken, so daß mit jeder Apertur 19-1 bis 19-4 eine eigene Messung ausgeführt werden kann, sind vorzugsweise streifenförmige Dichtungeinrichtungen 23 und 24 vorgesehen. Die Dichtungeinrichtungen 23 weisen die Form eines die freiliegende Fläche des Filterteiles 18 in vier Bereiche unterteilenden Kreuzes auf, während die Dichtungeinrichtung 24 die Form eines konzentrisch zu dem Kreis, auf dem die Aperturen 19-1 bis 19-4 liegen, angeordneten Kreises besitzt. In jeder der gebildeten Teilflächen 18-1 bis 18-4 des Filterteiles 18 ist eine Apertur 19-1 bis 19-4 von den jeweils anderen Aperturen isoliert angeordnet. Es ist auch denkbar, um jede Apertur 19-1 bis 19-4 eine einzige kreisförmige Dichteinrichtung anzuordnen, wie dies am Beispiel der Apertur 19-4 in der Figur 4 durch eine punktierte Linie dargestellt ist. Entsprechend der Verteilung der Aperturen 19-1 bis 19-4 befinden sich in dem Kapillarenhalter 9 an der dem Aperturhalter 3 zugewandten Fläche Vertiefungen 20-1 bis 20-4 derart, daß beim Aufliegen des Aperturhalters 3 auf der ihm zugewandten Fläche des Kapillarenhalters 9 jeweils eine der Vertiefungen eine Apertur umgibt. In der dargestellten Weise ist jede Vertiefung 20-1 bis 20-4 mit einer Kapillare 15-1 bis 15-4 verbunden. In der aus der Figur 5 ersichtlichen Weise, die eine Aufsicht auf den Kapillarenhalter 9 von unten zeigt, verlaufen die Kapillaren 15-1 bis 15-4 ausgehend von den Vertiefungen 20-1 bis 20-4 nach unten aufeinander zu, so daß ihre benachbarten Enden in ein Vorratsgefäß 5 eintauchen können.

In der entsprechenden Weise sind in der dem Aperturhalter 3 zugewandten Fläche des Kopfteiles 10 Vertiefungen 21-1 bis 21-4 vorgesehen, von denen jeweils eine im geschlossenen Zustand des Prüfkopfes 7 eine Apertur 19-1 bis 19-4 umgibt. In diesem Zusammenhang wird darauf hingewiesen, daß die zuvor beschriebenen Dichtungeinrichtungen 23, 24 bzw. die in der Figur 4 gepunktet dargestellten Dichtungeinrichtungen auf beiden Seiten des Aperturhalters 3 vorgesehen sind, so daß auch die Vertiefungen 21-1 bis 21-4 gegeneinander abgedichtet sind. Jede der Vertiefungen 21-1 bis 21-4 ist mit einer Leitung 11-1 bis 11-4 verbunden, die zu dem bereits erläuterten Zylinder 12 führen.

In der beschriebenen Anordnung stellt jede Kapillare 15-1 bis 15-4 im Zusammenhang mit einer Vertiefung 20-1 bis 20-4, einer Apertur 19-1 bis 19-4, einer Vertiefung 21-1 bis 21-4 und einer Leitung 11-1 bis 11-4 ein eigenes Prüfsystem dar. Es wird ausdrücklich darauf hingewiesen, daß das Filterteil 18 des Aperturhalters 3 sowohl eine als auch mehrere Aperturen 19 in einer beliebigen Zahl und Anordnung aufweisen kann, sofern dafür Sorge getragen ist, daß jede Apertur im Zusammenhang mit den beschriebenen Dichtungselementen, Kapillaren und Vertiefungen des Kapillarenhalters 9 und des Kopfteiles 10 ein eigenens Meßsystem darstellen kann.

Bei der Vorsehung mehrerer Meßsysteme können entweder mit den einzelnen Meßsystemen unterschiedliche Messungen vorgenommen werden, oder es können mit mehreren Meßsystemen, wenigstens mit zwei Meßsystemen, dieselben Messungen ausgeführt werden. Im zuletzt genannten Fall kann die Meßsicherheit wesentlich erhöht werden. Zur Vornahme unterschiedlicher Messungen können die Aperturen 19-1 bis 19-4 zumindest teilweise unterschiedlich groß sein. Vor dem Schließen des Prüfkopfes 7, das vorzugsweise durch Bewegen des Kopfteiles 10 gegen den feststehenden Kapillarenhalter 9, auf dessen Fläche 9' der Aperturhalter 3 angeordnet ist, erfolgt, kann durch eine geeignete Einrichtung in der Richtung des Pfeiles 25 automatisch ADP in die einzelnen gegeneinander abgedichteten Filterflächen 18-1 bis 18-3 eingegeben werden, so daß die genannten Filterflächen mit ADP getränkt werden.

In der Figur 2 stellen die Bezugszeichen 26 und 27 eine Reinigungsvorrichtung dar, mit deren Hilfe nach der Durchführung einer Messung beispielsweise das Vorratsgefäß 5 zur Reinigung durch Zugabe eines Spülmittels über die Leitung 26 aus dem Reservoir 27 gespült werden kann, wobei danach das Spülmittel durch die Kapillaren 15-1 bis 15-4 zur Reinigung der Vertiefungen 20-1 bis 20-4 und 21-1 bis 21-4 eingesaugt und wieder ausgestoßen werden kann. Zu diesem Zweck können die Leitungen 11-1 bis 11-4 durch nicht dargestellte Ventile vom Zylinder 12 abgetrennt und mit einer Druck- bzw. Unterdruckquelle (ebenfalls nicht dargestellt) verbunden werden. Es kann auch in einem eigenen Schritt nach dem Entfernen eines gerade geprüften Vorratsgefäßes ein das Spülmittel enthaltendes Gefäß in den Bereich der Kapillaren gebracht werden.

Es wird darauf hingewiesen, daß bei einer besonders bevorzugten, kostengünstigen Ausführungsform in den Randbereichen der Vertiefungen 20-1 bis 20-4 und 21-1 bis 21-4 Dichtungsringe 28 derart gehalten sind, daß die Isolierung der einzelnen Vertiefungen und Aperturen gegeneinander beim Schließen des Prüfkopfes dadurch erfolgt, daß die Dichtungen 28 gegen das Filterteil 18 gedrückt werden, wobei jeweils ein Dichtungsring 28 eine Apertur 19-1 bis 19-4 umgibt. In diesem Fall brauchen die Aperturhalter 3 die erwähnten Dichtungselemente 23 und 24 nicht aufweisen, so daß sie besonders kostengünstig herstellbar sind.

Im folgenden werden im Zusammenhang mit der Figuren 6 und 7 beispielhafte, besonders bevorzugte Einrichtungen erläutert, mit deren Hilfe aufeinanderfolgend Prüfoperationen durchgeführt werden können, wobei gleichzeitig mit einer Prüfoperation nach Entfernen eines Aperturhalters eine Spüloperation ausgeführt werden kann.

In der Figur 6 ist ein Drehteller 30 dargestellt, dem in einer ersten Stellung zur Zeit t₁ von einer Magazineinrichtung 2 bzw. 2' auf dem Wege 32 ein Aperturhalter 3 zu einem Ort I zugeführt wird. Dabei wird der Aperturhalter 3 in einer vorgegebenen Position auf den Drehteller 30 angeordnet. Von dem Ort I beabstandet befindet sich am Ort II der Prüfkopf 7, an dem zur Zeit t₁ der Bestückung des Ortes I mit einem Aperturhalter 3 gerade eine Messung an einem anderen Aperturhalter 3 ausgeführt wird. Am Ort III befindet sich zu dieser Zeit ein bereits geprüfter Aperturhalter 3', der zu dieser Zeit auf dem Wege 33 von dem Drehteller 30 abgenommen wird.

Zur Zeit t₂ wird des Drehteller 30 in der Drehrichtung 31 derart gedreht, daß der am Ort I befindliche Aperturhalter 3 zu einem Ort zwischen den Orten I und II gelangt und daß der Aperturhalter 3 am Ort II aus dem Prüfkopf 7 zu einem Ort zwischen den Orten II und III gedreht wird. Während der Drehteller 30 in dieser Stellung verweilt, wird der Prüfkopf 7 geschlossen und es wird in der im Zusammenhang mit der Figur 2 erläuterten Weise eine Spüloperation ausgeführt.

Nach Beendigung der Spüloperation wird der Prüfkopf 7 wieder geöffnet und zum Zeitpunkt t₃ des Drehteller 30 in der Richtung 31 derart gedreht, daß der zwischen den Orten I und II befindliche Aperturhalter 3 in den Prüfkopf 7 gelangt und der zwischen den Orten II und III befindliche Aperturhalter 3', an dem bereits eine Messung ausgeführt wurde, an den Ort III gelangt. Der Prüfkopf 7 wird dann wieder geschlossen und es wird erneut eine Messung ausgeführt. Gleichzeitig wird zum Ort I aus der Magazineinrichtung 2, 2' wieder ein neuer Aperturhalter 3 zugeführt, während vom Ort III der Aperturhalter 3' entnommen wird.

In der dargestellten Weise sind die Orte I, II und III auf dem Drehteller 30 beispielsweise um 90 ° voneinander beabstandet.

Aus der Figur 7 geht eine Weiterbildung der vorliegenden Einrichtung hervor, bei der die Aperturhalter 3 auf einem Schieber 40 angeordnet werden, der in seiner einen Position (obere Darstellungen der Figur 7) zusammen mit dem Aperturhalter 3 in den geöffneten Prüfkopf 10 eingeschoben ist und der in seiner anderen Stellung (gepunktete Darstellung der Figur 7) in Richtung des Pfeiles 41 aus dem Prüfkopf 10 herausgefahren ist. In dieser anderen Stellung wird der Prüfkopf 10 geschlossen und in der bereits beschriebenen Weise gereinigt. Gleichzeitig wird der Aperturhalter 3, an dem zuvor eine Messung ausgeführt wurde, vom Schieber 40 entfernt und aus der Magazineinrichtung 2 bzw. 2' ein neuer Aperturhalter 3 auf den Schieber 40 aufgebracht.

Die Ausführungsform der Figur 7 ist besonders vorteilhaft, weil, wie dies ersichtlich ist, zur Raumsparung die vorzugsweise rechteckig ausgebildeten Prüfköpfe 10 unmittelbar nebeneinander angeordnet werden können.

Bei den Ausführungsformen der Figuren 6 und 7 können die Vorratsgefäße ebenfalls durch eine weitere Transportscheibe bzw. einen weiteren Schieber zugeführt werden, wobei die Bewegungen der weiteren Transportscheibe bzw. des weiteren Schiebers und die Bewegungen der Transportscheibe 30 bzw. des Schiebers 40 z.B. durch mechanische Einrichtungen synchronisiert sein können.

Es wird darauf hingewiesen, daß die schematische Darstellung der Figur 2 so zu verstehen ist, daß mit jeder Leitung 11-1 bis 11-4 etc. ein eigener Zylinder 12 verbunden ist, in dem sich ein Kolben 13 befindet.

Es ist denkbar, das Vorratsgefäß 5 durch wenigstens eine Trennwand 5" (siehe Figur 1) in mehrere Teilräume zu unterteilen, wobei jedem Teilraum eine Kapillare 15 zur Blutentnahme zugeordnet ist.

Unter "Filterteil" wird ein Teil verstanden, das aus einem porösen Teil (z.B. einem Filterteil oder aus Kollagen selbst) oder einer Membran besteht und mit einem undurchlässigen Material abgestützt ist. In dem porösen Teil können eine oder auch mehrere Aperturen angeordnet sein.

## Patentansprüche

1. Einrichtung zur automatischen Untersuchung von Blutproben mit einer Prüfstation (1), in der Blut aus einem Vorratsgefäß (5) entnommen und durch eine Apertur (19) in einem in einem Aperturhalter (3) gehaltenen Teil (18) hindurchgeführt wird, wobei
wenigstens ein Prüfkopf (7) vorhanden ist, dem gleichzeitig aus einer ersten Magazineinrichtung (8) Vorratsgefäße (5) mit Blut und aus einer weiteren Magazineinrichtung (2; 2') Aperturhalter (3) zugeführt werden, wobei
der Prüfkopf (7) einen Kapillarenhalter (9), an dem wenigstens eine in ein Vorratsgefäß (5) einführbare Kapillare (15) befestigt ist, und ein Kopfteil (10) aufweist, das durch Relativbewegung zum Kapillarenhalter (9) zwischen einer ersten Position, in der ein Aperturhalter (3) auf dem Kapillarenhalter (9) angeordnet wird, und einer zweiten Position bewegt wird, in der der Aperturhalter (3) zwischen dem Kapillarenhalter (9) und dem Kopfteil (10) so gehalten wird, daß über die in ein Vorratsgefäß (5) eintauchende Kapillare (15) Blut aus dem Vorratsgefäß (5) durch die Kapillare (15) und die Apertur (19) des Aperturhalters (3) hindurchführbar ist, und wobei
jeweils nach Vornahme wenigstens einer Messung an einem Aperturhalter (3) unter Hindurchführung von Blut der benutzte Aperturhalter (3) und das benutzte Vorratsgefäß (5) nach Bewegung des Kopfteiles (10) in die erste Position des Kopfteiles (10) aus dem Prüfkopf (7) entfernt wird und danach der Prüfkopf (7) durch eine Reinigungsvorrichtung (26, 27) gereinigt wird.

2. Einrichtung nach Anspruch 1, wobei der Aperturhalter (3) die Form eines platten- bzw. scheibenförmigen Teiles (16) aufweist, an dem ein die Apertur (19) aufweisendes Teil (18) gehalten ist.

3. Einrichtung nach Anspruch 2, wobei das Teil ein scheibenförmiges Filterteil (18) ist, das eine Öffnung (17) in dem plattenförmigen Teil (16) überdeckt.

4. Einrichtung nach Anspruch 3, wobei das Filterteil (18) zwischen zwei plattenförmigen Teilen (16, 16) angeordnet ist, deren Öffnungen (17, 17) zueinander ausgerichtet sind.

5. Einrichtung nach einem der Ansprüche 1 bis 4, wobei die zweite Magazineinrichtung (2) die Form einer Vorratsspule aufweist, auf der die bandförmig miteinander verbundenen Aperturhalter (3) aufgewickelt sind.

6. Einrichtung nach einem der Ansprüche 1 bis 4, wobei die zweite Magazineinrichtung (2') zu einen Stapel übereinander angeordnete Aperturhalter (3) speichert.

7. Einrichtung nach einem der Ansprüche 1 bis 6, wobei aus der ersten Magazineinrichtung (8) die in einer Halterung befindlichen Vorratsgefäße (5) hintereinander zu dem Prüfkopf (1) geführt werden.

8. Einrichtung nach einem der Ansprüche 1 bis 7, wobei der Kapillarenhalter (9) in der dem Kopfteil (10) zugewandten Fläche (9') eine Vertiefung (20) aufweist, die mit der Kapillare (15) in Verbindung steht und die in der zweiten Position des Prüfkopfes (7) durch das die Apertur (19) aufweisende Teil (18) oder durch den Aperturhalter (3) dicht verschlossen ist, so daß sich die Apertur (19) im Bereich der Vertiefung (20) des Kapillarenhalters (9) befindet.

9. Einrichtung nach Anspruch 8, wobei das Kopfteil (10) in der dem Kapillarenhalter (9) zugewandten Fläche (10') eine Vertiefung (21) aufweist, die mit einer Leitung (11) in Verbindung steht, die mit einem Zylinder (12) einer Meßeinrichtung verbunden ist, in dem durch die Bewegung eines Kolbens (13) ein Unterdruck erzeugbar ist, wobei die Vertiefung (21) des Kopfteiles (10) in der zweiten Position des Prüfkopfes (7) durch das die Apertur (19) aufweisende Teil (18) oder den Aperturhalter (3) dicht verschlossen ist, derart, daß sich die Apertur (19) im Bereich der Vertiefung (21) des Kopfteiles (10) befindet.

10. Einrichtung nach Anspruch 9, wobei die Vertiefungen (20, 21) des Kapillarenhalters (9) und des Kopfteiles (10) deckungsgleich zueinander ausgerichtet sind.

11. Einrichtung nach Anspruch 9 oder 10, wobei an dem Aperturhalter (3) an beiden Seiten eine die Apertur (19) ringartig umgebende Dichtungseinrichtung (22; 23, 24) derart vorgesehen ist, daß in der zweiten Position des Prüfkopfes (7) der Randbereich der Vertiefungen (20, 21) des Kapillarenhalters (9) und des Kopfteiles (10) abgedichtet sind.

12. Einrichtung nach Anspruch 9 oder 10, wobei die Randbereiche der Vertiefungen (20, 21) des Kapillarenhalters (9) und des Kopfteiles (10) mit ringartigen Dichtungseinrichtungen (28) versehen sind, die in der zweiten Position des Prüfkopfes (7) den Bereich der Apertur (19) dicht umschließen.

13. Einrichtung nach einem der Ansprüche 1 bis 12, wobei jeweils in dem Kapillarenhalter (9) und dem Kopfteil (10) mehrere Vertiefungen (20-1 bis 20-4, 21-1 bis 21-4) und in dem Teil (18) des Aperturhalters (3) mehrere Apertuen (19-1 bis 19-4) angeordnet sind, wobei jeweils eine Apertur einer Vertiefung des Kapillarenhalters (9) und einer Vertiefung (21-1 bis 21-4) des Kopfteiles (10) zugeordnet ist, wobei jede Vertiefung (20-1 bis 20-4) des Kapillarenhalters (9) mit einer Kapillare (15-1 bis 15-4) verbunden ist und wobei jede Vertiefung des Kopfteiles (10) mit der Meßeinrichtung in Verbindung steht.

14. Einrichtung nach einem der Ansprüche 1 bis 13, wobei in der zweiten Position zur Spülung nach der Durchführung einer Messung ohne die Vorsehung eines Aperturhalters (3) die Kapillare (15) und die Vertiefungen (20, 21) mit einem von der Reinigungsvorrichtung (26, 27) zugeführten Spülmittel ausgewaschen werden.

15. Einrichtung nach einem der Ansprüche 1 bis 14, wobei ein Schieber (40) vorhanden ist, der in der ersten Position einen Aperturhalter (3) in den Prüfkopf (7) einschiebt und nach der Ausführung einer Messung aus dem Prüfkopf (7) herausbewegt und wobei dem herausbewegtem Schieber (40) die Aperturhalter (3) von der Magazineinrichtung (2; 2') zugeführt werden und vom herausbewegten Schieber (40) die benutzten Aperturhalter (3') abgenommen werden.

16. Einrichtung nach Anspruch 15, wobei mehrere Schieber (40) nebeneinander angeordnet sind.

17. Einrichtung nach einem der Ansprüche 1 bis 14, wobei die Aperturhalter (3) einem Ort (I) eines durch den Prüfkopf (7) verlaufenden Drehtellers (30) aufeinanderfolgend zugeführt werden, der in der ersten Position des Prüfkopfes (7) in den Prüfkopf (7) gedreht wird und nach einer Messung in der ersten Position des Prüfkopfes (7) aus dem Prüfkopf (7) herausbewegt wird, so daß die Abnahme des benutzten Aperturhalters (3') vom Drehteller (30) erfolgen kann.

## Claims

1. An apparatus for the automatic testing of blood samples having a test station (1) in which blood is taken from a supply vessel (5) and is passed through an aperture (19) in a part (18) held in a aperture holder (3), wherein
at least one test head (7) is provided, which is supplied simultaneously with supply vessels (5) containing blood from a first magazine device (8) and aperture holders (3) from a further magazine device (2; 2'), wherein
the test head (7) comprises a capillary tube holder (9), to which at least one capillary tube (15) which can be introduced into a supply vessel (5) is attached, and a head part (10), which by the relative movement to the capillary tube holder (9) is disposed between a first position, in which an aperture holder (3) is disposed on the capillary tube holder (9), and a second position, in which the aperture holder (3) is kept between the capillary tube holder (9) and the head part (10), so that via the capillary tube (15) dipping into a supply vessel (5) blood from the supply vessel (5) can be conveyed through the capillary tube (15) and the aperture (19) of the aperture holder (3), and wherein
in each case after the performance of at least one measurement at an aperture holder (3) with the passage of blood, the used aperture holder (3) and the used storage vessel (5) is removed from the test head (7) after a movement of the head part (10) into the first position of the head part (10) and then the test head (7) is cleaned by a cleaning device (26, 27).

2. An apparatus according to Claim 1, wherein the aperture holder (3) has the form of a plate-shaped or disc-shaped part (16) on which a part (18) having the aperture (19) is retained.

3. An apparatus according to Claim 2, wherein the part is a disc-shaped filter part (18) which covers an opening (17) in the plate-shaped part (16).

4. An apparatus according to Claim 3, wherein the filter part (18) is disposed between two plate-shaped parts (16, 16), the openings (17, 17) of which are aligned with one another.

5. An apparatus according to one of Claims 1 to 4, wherein the second magazine device (2) has the form of a supply reel on which are wound the aperture holders (3) which are connected to one another in a strip.

6. An apparatus according to one of Claims 1 to 4, wherein the second magazine device (2') stores aperture holders (3) which are disposed one on top of another in a pile.

7. An apparatus according to one of Claims 1 to 6, wherein from a first magazine device (8) the storage vessels (5) situated in a mounting are conveyed one behind the other to the test head (1).

8. An apparatus according to one of Claims 1 to 7, wherein the capillary tube holder (9) in the face (9') directed towards the head part (10) has a cavity (20), which communicates with the capillary tube (15) and which in the second position of the test head (7) is tightly closed by the part (18) having the aperture (19) or by the aperture holder (3), so that the aperture (19) is situated in the region of the cavity (20) of the capillary tube holder (9).

9. An apparatus according to Claim 8, wherein the head part (10) in the face (10') directed towards the capillary tube holder (9) has a cavity (21) which communicates with a pipe (11), which is connected with a cylinder (12) of a measuring instrument in which by the movement of a plunger (13) low pressure is produced, wherein the cavity (21) of the head part (10) in the second position of the test head (7) is tightly closed by the part (18) comprising the aperture (19) or by the aperture holder (3) in such a manner that the aperture (19) is situated in the region of the cavity (21) of the head part (10).

10. An apparatus according to Claim 9, wherein the cavities (20, 21) of the capillary tube holder (9) and of the head part (10) are aligned congruently.

11. An apparatus according to Claim 9 or 10, wherein provided at the aperture holder (3) at both sides is a sealing device (22; 23, 24) surrounding the aperture (19) in the manner of a ring in such a manner that in the second position of the test head (7) the edge region of the cavities (20, 21) of the capillary tube holder (9) and of the head part (10) are sealed.

12. An apparatus according to Claim 9 or 10, wherein the edge regions of the cavities (20, 21) of the capillary tube holder (9) and of the head part (10) are provided with ring-shaped sealing devices (20), which in the second position of the test head (7) tightly surround the region of the aperture (19).

13. An apparatus according to one of Claims 1 to 12, wherein disposed in the capillary tube holder (9) and the head part (10) respectively are several cavities (20-1 to 20-4, 21-1 to 21-4) and disposed in the part (18) of the aperture holder (3) are several apertures (19-1 to 19-4), wherein one aperture respectively is associated with one cavity of the capillary tube holder (9) and with one cavity (21-1 to 21-4) of the head part (10), wherein each cavity (20-1 to 20-4) of the capillary tube holder (9) is connected to a capillary tube (15-1 to 15-4) and wherein each cavity of the head part (10) communicates with the measurement instrument.

14. An apparatus according to one of Claims 1 to 13, wherein in the second position for rinsing after the performance of a measurement without the provision of an aperture holder (3) the capillary tubes (15) and the cavities (20, 21) are rinsed out with a rinsing medium supplied from the cleaning device (26, 27).

15. An apparatus according to one of Claims 1 to 14, wherein a slide (40) is provided, which in the first position inserts an aperture holder (3) into the test head (7) and after the performance of a measurement moves it out of the test head (7) and wherein the aperture holder (3) is supplied from the magazine device (2; 2') to the moved-out slide (40) and the used aperture holders (3') are removed from the moved-out slide (40).

16. An apparatus according to Claim 15, wherein several slides (40) are disposed next to one another.

17. An apparatus according to one of Claims 1 to 14, wherein the aperture holders (3) are supplied to a location (I) of a rotary table (30) extending through the test head (7), which table, in the first position of the test head (7), is rotated into the test head (7) and after a measurement in the first position of the test head (7) is moved out of the test head (7) so that the removal of the used aperture holder (3') from the rotary table (30) can take place.

## Revendications

1. Dispositif pour l'analyse automatique d'échantillons de sang comprenant une station d'analyse (1) dans laquelle du sang est prélevé dans un récipient (5) et passe à travers un orifice (19) dans un élément (18) qui est tenu dans un support à orifices (3), dans lequel
il est prévu au moins un tête d'analyse (7) à laquelle sont amenés simultanément des récipients (5) avec du sang provenant d'un premier dispositif formant magasin (8) et des supports à orifices (3) provenant d'un autre dispositif formant magasin (2, 2')
la tête d'analyse (7) comprend un support à capillaires (9) auquel est fixé au moins capillaire (15) qui peut être introduit dans un récipient (5) et une partie tête (10) qui est amenée par déplacement relatif par rapport au support à capillaires (9) d'une première position, dans laquelle un support à orifices (3) est disposé sur le support à capillaires (9), dans une seconde position, dans laquelle le support à orifices (3) est tenu entre le support à capillaires (9) et la partie tête (10), de manière telle que du sang puisse être amené, via le capillaire (15) qui plonge dans un récipient (5), dans le capillaire (15) et dans l'orifice (19) du support à orifices (3),
et, après la réalisation d'au moins une mesure à l'aide d'un support a orifices (3) impliquant le passage de sang, le support à orifices (3) utilisé et le récipient (5) utilisé, après déplacement de la partie tête (10) dans la première position de ladite partie tête (10), sont éloignés de la tête d'analyse (7) et la tête d'analyse (7) est nettoyée par un dispositif de nettoyage (26, 27).

2. Dispositif selon la revendication 1, dans lequel le support à orifices (3) est réalisé sous la forme d'un élément (16) en forme de plaque ou de disque sur lequel un élément (18) portant l'orifice (19) est tenu.

3. Dispositif selon la revendication 2, dans lequel l'élément est un élément de filtre (18) en forme de disque qui recouvre une ouverture (17) dans l'élément (16) en forme de plaque.

4. Dispositif selon la revendication 3, dans lequel l'élément de filtre (18) est disposé entre deux éléments (16, 16) en forme de plaques dont les ouvertures sont mutuellement alignées.

5. Dispositif selon une des revendications 1 à 4, dans lequel le deuxième dispositif formant magasin (2) se présente sous la forme d'une bobine de stockage sur laquelle sont enroulés les supports à orifices (3) liés les uns aux autres en une bande.

6. Dispositif selon une des revendications 1 à 4, dans lequel le deuxième dispositif formant magasin (2) stocke les supports à orifices (3) les uns au-dessus des autres, en une pile.

7. Dispositif selon une des revendications 1 à 6, dans lequel les récipients (5) qui se trouvent dans un support dans le premier dispositif formant magasin (8) sont amenés les uns à la suite des autres à la tête d'analyse (1).

8. Dispositif selon une des revendications 1 à 7, dans lequel le support à capillaires (9), dans sa surface (9') tournée vers la partie tête (10), présente une cavité (20) qui communique avec le capillaire (15) et qui, dans la deuxième position de la tête d'analyse (7), est fermée de manière étanche par l'élément (18) comportant l'orifice (19) ou par le support à orifices (3) de sorte que l'orifice (19) se trouve dans dans la région de la cavité (20) du support à capillaires (9).

9. Dispositif selon la revendication 8, dans lequel la partie tête (10), dans sa surface (10') tournée vers le support à capillaires (9), présente une cavité (21) qui est reliée à une conduite (11) qui communique avec un cylindre (12) d'un dispositif de mesure à l'intérieur duquel une dépression peut être générée par déplacement d'un piston (13), la cavité (21) de la partie tête (10), dans la deuxième position de la tête d'analyse (7), étant fermée de manière étanche par l'élément (18) pourvu de l'orifice (19) ou par le support à orifices (3) de telle sorte que l'orifice (19) se trouve dans la région de la cavité (21) de la partie tête (10).

10. Dispositif selon la revendication 9, dans lequel les cavités (20, 21) du support à capillaires (9) et de la partie tête (10) sont parfaitement alignées l'une avec l'autre.

11. Dispositif selon la revendication 9 ou 10, dans lequel il est prévu sur le support à orifices (3), sur les deux faces, un dispositif de joint (22; 23, 24) qui entoure à la manière d'un anneau l'orifice (19) de telle sorte que dans la deuxième position de la tête d'analyse (7) la zone bord des cavités (20, 21) du support à capillaires (9) et de la partie tête (10) soit fermée de manière étanche.

12. Dispositif selon la revendication 9 ou 10, dans lequel les zones de bord des cavités (20, 21) du support à capillaires (9) et de la partie tête (10) sont munis de dispositifs de joint (28) annulaires qui entourent de manière étanche la région de l'orifice (19) dans la deuxième position de la tête d'analyse (7).

13. Dispositif selon une des revendications 1 à 12, dans lequel plusieurs cavités (20-1 à 20-4, 21-1 à 21-4) sont aménagées chaque fois dans le support à capillaires (9) et la partie tête (10), et plusieurs cavités (19-1 à 19-4) sont aménagées dans l'élément (18) du support à orifices (3), dans lequel un orifice est associé chaque fois à une cavité du support à capillaires (9) et une cavité (21-1 à 21-4) de la partie tête (10), dans lequel chaque cavité (20-1 à 20-4) du support à capillaires (9) est connectée à un capillaire (15- à 15-4) et dans lequel chaque cavité de la partie tête (10) est reliée au dispositif de mesure.

14. Dispositif selon une des revendications 1 à 13, dans lequel, dans la deuxième position pour le lavage après réalisation d'une mesure sans support à orifices (3), les capillaires (15) et les cavités (20, 21) sont lavées à l'aide d'un produit de lavage envoyé par le dispositif de nettoyage (26, 27).

15. Dispositif selon une des revendications 1 à 14, dans lequel il est prévu un tiroir (40) qui, dans la première position, glisse un support à orifices (3) dans la tête d'analyse (7) et, après une mesure, éjecte ledit support à orifices de la tête d'analyse (7) et dans lequel les supports à orifices (3) sont chargés sur le tiroir (40) sorti à partir du dispositif formant magasin (2; 2') et les supports à orifices (3) sont retirés du tiroir (40) sorti.

16. Dispositif selon la revendication 15, dans lequel plusieurs tiroirs (40) sont disposés les uns à côté des autres.

17. Dispositif selon une des revendications 1 à 14, dans lequel les supports à orifices (3) sont amenés successivement en un point (I) d'un plateau tournant (30) qui passe par la tête d'analyse (7), lequel plateau, dans la première position de la tête d'analyse (7) est amené par rotation dans la tête d'analyse (7) et après une mesure dans la première position de la tête d'analyse (7) est éloigné de ladite tête d'analyse (7) de telle sorte que le support à orifices (3') utilisé puisse être déchargé du plateau tournant (30).
